# EUROPEAN PATENT SPECIFICATION

(11) **EP 1 853 229 B1**
(45) Date of publication and mention of the grant of the patent: **25.03.2015**
(21) Application number: 06708308.9
(22) Date of filing: 16.02.2006
(51) Int. Cl.: A61K 9/20, C02F 1/76

(54) **COMPOSITION FOR ADJUSTING WATER QUALITY FOR ADMINISTRATION OF WATER SANITIZER SENSITIVE MEDICAMENTS OR VACCINES**
ZUSAMMENSETZUNG ZUR ANPASSUNG DER WASSERQUALITÄT ZUR VERABREICHUNG VON MEDIKAMENTEN ODER IMPFSTOFFEN, DIE AUF WASSERDESINFEKTIONSMITTEL EMPFINDLICH REAGIEREN
COMPOSITION PERMETTANT D'AJUSTER LA QUALITE DE L'EAU POUR ADMINISTRATION DE MEDICAMENTS OU DE VACCINS SENSIBLES A UN STERILISATEUR D'EAU

(30) Priority: 18.02.2005 BR PI0500660
(43) Date of publication of application: 14.11.2007
(73) Proprietor: Salmix Industria e Comercio LTDA, 170-000 Sao Paulo (BR); Intervet International B.V., 5831 AN Boxmeer (NL)
(72) Inventor: RAVETTI, Renato, Piedade, 170-000 Sao Paulo (BR)
(74) Representative: Stumm, Karin
(86) International application number: PCT/EP2006/060014
(87) International publication number: WO 2006/087358

(56) References cited:
- DE-A1- 3 632 334
- GB-A- 1 002 264
- GB-A- 1 024 408
- GB-A- 1 221 038
- US-A- 466 610

## Description

The present invention relates to a composition for adjusting water quality for administration of water sanitizer sensitive medicaments or vaccines.

Over the past several years, the farm size in livestock animals has been increasing. To cope with the increased farm size, animal caretakers now require mass vaccination of animals via spray and drinking water over individual inoculation by injection. This mass administration of vaccine by aerosol spray or drinking water benefits those producing the animals by reducing labor and eliminating the injection site injuries and broken needle residue that threatens the quality and safety of meat products.

In modern industrial aviculture water, besides being an essential nutrient, exercises an important role as carrier of medicaments for therapeutic or preventive use and for imunoprophylactic programs such as vaccination and immunization.

A number of attenuated live vaccines for oral administration e.g. via the drinking water or for spray administration for poultry are available commercially e.g. to protect against diseases such as Avian Encephalomyelitis, Infectious Bursal Disease (Gumboro), Newcastle Disease, Infectious Bronchitis, Avian Laryngotracheitis (LT), Avian Pneumovirus infections or Salmonellosis.

Poultry and other animal industry productivity improvements are related to all production factors, but water quality is particularly important. In the field, many different water sources can be used for animal consumption, such as water wells, fountains, shallow wells, semi-artesian and artesian wells, lakes and creeks. The use of these water sources exposes the animals to several pathogenic virus and bacteria through contamination by wild animals. It is therefore necessary to sanitize such water sources by adding oxidizing sanitizers such as chlorine, peroxide, bromine, and the like to water.

A review of the main methodologies used for water sanitization indicates that chlorination is the most adequate technology for animal caretakers with the objective of treating the water in light of contamination and in fact, chlorination eliminates viruses and bacteria. In general, a level of residual chlorine between 2 and 3 ppm at the most distant point of its introduction in the water system is recommended.

While these sanitizers disinfect the water of common pathogenic organisms, they also kill the infectious agents present in live vaccines when they are administered through drinking water or such water is used to prepare aerosol spray. The result is a complete loss of the vaccine's potency and failure to protect the animals from subsequent infections.

In case of vaccines with live viruses, the presence of a small amount of chlorine will cause a partial or complete deactivation of the vaccine virus and without the proper number of living viruses, a complete immunization cannot be developed. In order to measure this effect the virus concentration can be determined.

The virus concentration on a suspension is expressed by the Infectivity Titre, which can be determined by making a vaccine titration. The unit of measuring infectivity of avirulent virus corresponds to 50% of the Embryo Infectious Dose. One EID₅₀ unit corresponds to the amount of vaccine virus that will infect 50% of inoculated eggs. The effect of the residual chlorine concentration in the water over the titre of a vaccine against Newcastle Disease is presented through the reduction of the EID₅₀ (Poultry Science, Vol 51, 1972, pp. 1450-1456).

**Table 1**

| Chlorine Concentration (ppm) | EID₅₀ (log₁₀) |
|---|---|
| 0 | 7.0 |
| 1 | 2.0 |
| 2.5 | 1.1 |
| 5 | 1.0 |
| 15 | 0.1 |
| 25 | 0 |

To solve this problem, it is one option, that chlorinators as well as other water sanitizers must have their administration interrupted 24 hours before the administration of the vaccine, during the administration and 24 hours after its administration in order to ensure that no (or only uncritical amounts of) such sanitizer are present at the drinking water during the time of administration of the vaccine or sanitizer sensitive medicament.

There are other alternative, but also troublesome, solutions to reduce the chlorine amount in water and its negative effect on the vaccines. The less expensive method consists of stocking this water in a tank that has a large surface area at a temperature of approximately 30°C for a period of 48 through 72 hours. This method is obviously not practical in light of the fact that the reservoir must have a large area and thus favor contamination of the water (e.g. by other birds).

One of the alternatives, still much employed is the usage of powdered skim milk (P.S.M.), which may neutralize the residual chlorine existing in concentrations higher than 1 ppm. The process corresponds to adding 240 grams of powdered skim milk (P.S.M.) in 100 liters of water. The protein fraction of powdered skim milk (P.S.M.) prevents the loss of infectivity occurring when the live vaccine is mixed to water with chlorine. Taking into account the increase of the concern with use of animal origin products, the powdered skim milk (P.S.M.) becomes a problem mainly for the exporting countries, that is, making this alternative unfeasible. Besides that, the powdered skim milk (P.S.M.) contaminates the drinking pond with some ingredients that make the system liable to the development of microorganisms. Also, the powdered skim milk (P.S.M.) does not fully dissolve in the cold water and this undissolved milk powder collects in vaccine delivery systems, and dogs the spray nozzles and orifices of drinking water dispensers. The clogged vaccinating equipment is thereby prevented from functioning properly and fails to vaccinate the animals uniformly and thereby, failing to confer immunity to the entire group.

Another option for adjusting the water quality, especially by de-chlorination, is based on the addition of sulfur compounds, such as calcium or sodium sulfite, sodium thiosulfate, sodium sulfide and others. This is described e.g. in the International application WO 01/26622 A1, although are effective dechlorinating agents, can be toxic to both humans and animals. The addition of excess sulfite and sulfate chemicals to our water has always been a concern, because it has a negative effect of reducing the oxygen levels in water, causing the death of water organisms.

Hence there is no satisfactory solution available that is able to adjust the quality of drinking water for animals used for administering vaccines, medicaments, probiotics or any other treatment where the active ingredient is sensible to the sanitizer that must be nontoxic and avoids these drawbacks.

Furthermore, there are additional factors that can adversely affect a live vaccine's viability. These factors include pH excursions beyond optimum limits for the vaccine, and organic oxidizers, which include nitrites and less commonly sulfites and chloramines.

Therefore a need exists for a composition that, adjusts the water quality in case water sanitizers are present to allow the administration of water sanitizer sensitive medicaments or vaccines. Such a composition when added to water, will secure the infectivity of live viral, parasitic and bacterial vaccine by reducing negative water quality factors that limit the life of the vaccine.

Such a composition will permit farms or animal caretakers to use their own integral water supply as a functional delivery vehicle for the vaccine and ultimately afford the animals greater protection from disease. Most important is that these products are safe and less toxic for animals and the environment than currently used products.

The current invention provides such a composition for adjusting water quality for administration of water sanitizer sensitive medicaments or vaccines characterized in that it comprises a natural reducing agent, a buffer and a coloring agent.

Water sanitizer sensitive medicaments or vaccines are e.g. live vaccines. Live vaccines are vaccines prepared from living attenuated organisms (bacteria, parasites and virus) or from viruses that have been attenuated but can still replicate the cells of the host organism. Live vaccines are living, avirulent forms of a micro-organism or virus that are used to elicit an antibody response that will protect the inoculated organism against infection by a virulent form of the micro-organism or virus. A live vaccine is also a living, avirulent microorganism or virus that express a foreign antigenic protein and is used to inoculate humans or animals. The latter organisms are also called a live recombinant vaccine.

Water sanitizer sensitive medicaments can be pharmaceutically active proteins, peptides and the like and chemical pharmaceutical compounds that are known to be degraded in the presence of water sanitizers. Examples of water sanitizers and other contaminants are chlorine, peroxide, bromine, fluorine, ozone, iodine, permanganate, chromic acid, chloramines, and nitrites.

A reducing agent is used in the composition to neutralize oxidizing sanitizers or contaminants present in the farm water. Natural reducing agents are for example ascorbic acid and its salts, especially alkali metal salts, tannic acid and tannins. The reducing agent is selected in an amount appropriate to neutralize the oxidizing sanitizers or contaminants present in the farm water that is to be used as the conveyance vehicle for the vaccine. Although the amount of reducing agent used in the composition of the present invention is at least about 40.0 percent by weight, it is more preferred that at least about 35.0 percent by weight be used and most preferred that at least about 30.0 percent by weight be used.

Preferably the natural agent is ascorbic acid or an alkali metal salt of ascorbic acid. Ascorbic acid and its salts react fast and stoichiometrically with the chlorine and have a limited interaction with disinfection byproducts. They completely neutralize chlorine and chloramines. There are several powerful arguments for using vitamins instead of sulfur-based compounds as dechlorination agents but the most important is that these products are safe and less toxic for animals and the environment. As a vitamin, we use the chemistry of the ascorbic acid for dechlorination. This treatment has presented to be efficient in neutralizing the residual chlorine present as HOCl, ClO⁻, and Cl₂. The ascorbic acid rapidly reduces the residual chlorine and has the advantage of producing an inorganic halide and the dehydroascorbic acid. The two forms, that is, the ascorbic acid or the dehydroascorbic acid can be interchangeable and both are biologically active inside the body. Before its absorption by the body, the ascorbic acid (reduced form) can be oxidized to dehydroascorbic acid, which is absorbed faster.

The concentrations used depend stoichiometrically on the concentrations of the oxidizing agents to be expected (chlorine and active chlorine compounds) and are in the range from 0.1-100 mg/l and preferably from 0.5 -20 mg/l.

Buffers are used to keep the pH of the water for administration generally in a range of about 6 - 7, which is appropriate for a spectrum of viral and bacterial antigens in live vaccines. The buffer preferably used in the composition is selected from citric acid and/ or alkali- or alkali bicarbonate. Other common buffers are phosphates, carboxylates, and bicarbonates. More preferred buffering agents are sodium phosphate, potassium phosphate, sodium ascorbate, sodium citrate, calcium lactate, sodium succinate, sodium glutamate, sodium bicarbonate, and potassium bicarbonate and / or citric and ascorbic acids. Although the amount of buffer used is from about 0.00 to about 98.0 percent by weight, it is preferred that from about 0.00 to about 80.0 percent by weight be used.

The composition according to the invention can be either a dry or a liquid form that is suitable for addition to tap or well water or similar diluent prior to the introduction of the vaccine. The state of the composition can be liquid or dry depending on the user's choice. Hence this invention contemplates both dry and liquid physical states of the composition. The dry embodiments contain ingredients appropriately selected, blended, and stored in a dry state to be dissolved in the vaccine vehicle when use is eminent. The liquid embodiments employ the use of less concentrated liquid formulations that contain a physiologically acceptable liquid diluent and carrier, preferably water. On occasion, the skilled artisan may deem it more suitable to his or her purpose to produce the composition according to the invention as a liquid preparation instead of a dry mixture. Such occasions would likely arise when the concentration and handling characteristics of a diluted liquid concentrate would lead to better measuring and mixing in the water vehicle. The dry state would be employed when higher concentrations of the composition are desired.

In the animal drinking system, the dry composition is e.g. dissolved in water to form a "stock solution", which is a premix of tap or farm well water, the composition according to the invention, and an appropriate amount of vaccine doses for the animal group. The composition is added first to neutralize the oxidizing sanitizers and contaminants, and makes the stock solution hospitable to the vaccine. The required doses of vaccine are then added to the stock solution, and the stock solution is further diluted in the animals' drinking water by a variety of means available to the caretaker. The composition present in the stock solution ensures that oxidizing sanitizers and contaminants in the greater volume of drinking water are also neutralized, rescuing the vaccine from potential decay. The animals drink the stock solution-laden water until all doses are consumed and all animals are vaccinated.

The composition further may comprise a coloring agent. This ingredient in the composition should be a water-soluble FD&C food color permitted for direct addition to human food that is preferably approved by the FDA, like the color additive FD&C Blue n° 1 that is principally the disodium salt of ethyl [4-[*p*-[ethyl (*m*-sulfobenzyl) amino]-α-(*o*-sulfophenyl) benzylidene] - 2,5 -cyclohexadien - 1 - ylidene] (*m-*sulfobenzyl) ammonium hydroxide inner salt with smaller amounts of the isomeric disodium salts of ethyl [4-[*p*-[ethyl(*p*-sulfobenzyl) amino]-α-(*o*-sulfophenyl) benzylidene]-2,5-cyclohexadien-1-ylidene] (*p*-sulfobenzyl) ammonium hydroxide inner salt and ethyl [4-[*p*-[ethyl (*o*-sulfobenzyl) amino]-α-(*o* -sulfophenyl) benzylidene]-2,5-cyclohexadien-1-ylidene] (*o*-sulfobenzyl) ammonium hydroxide inner salt. The coloring provides visual verification to the animal caretaker that the composition has been added to the water, and the stabilized water solution is prepared to receive the vaccine. Additionally, the colorant remains in the vaccine spray or drinking water to mark the feathers, skin, hair, wool, lips or tongues of the animals that have been sprayed or that have consumed the water. Such marking aids the health management of the animals by serving as a visual reference to the caretaker for positively identifying the vaccinated and non-vaccinated animals. Although the amount of coloring agent used in the composition of the present invention is from about 0.00 to about 35.0 percent by weight, it is preferred that at least about 8.50 percent by weight be used, more preferred that at least about 17.50 percent by weight be used, and most preferred that at least about 23.0 percent by weight be used.

In one embodiment the composition according to the invention is in a tablet form. In a specific embodiment the composition is in the form of an effervescent tablet.

One of the objectives of the present invention is to supply a process for preparing an effervescent tablet, soluble in water, without products of animal origin, stabilizing or neutralizing chiefly the residual chlorine and chloramines existent in the water used for administering vaccines, medicines, probiotics, or any other treatment where the active ingredient is sensible to the sanitizer, composed by a vitamin such as the ascorbic acid and its salts, that is, sodium or calcium ascorbate, sodium bicarbonate, citric acid, binder and lubricant for direct compression. The dechlorination agent is Vitamin C 100% pharmaceutical degree, the final formulation is safe for the environment, 100% organic and contains an essential nutrient for health.

Another objective of the present invention is to supply the animal with a nutrient, which will be available on its reduced form as ascorbic acid or on its oxidized form as dehydroascorbic acid, both biologically active and effectively contributing for the reduction of the stress caused by the vaccination process or application of a given medicine via drinking water. There are considerable evidences showing that the birds are not capable of synthesizing a sufficient amount of ascorbic acid to restore the amount loss during a stressing event, as for example, during the first five days of life or during vaccination. The amount of ascorbic acid supplied by the use of this tablet for water de-chlorination corresponds to 15% of the daily need, that is, the ascorbic acid besides neutralizing the sanitizer preserving the quality of the medicine to be ministered, contributes significantly for reducing a stress situation improving the efficiency of the medicine ministering process.

According to one invention preparation, the tablet has the following generic compound:
1 to 40% (w/w) of ascorbic acid
1 to 40% (w/w) of sodium bicarbonate
1 to 30% (w/w) of citric acid
1 to 35% (w/w) of a food coloring agent
1 to 20% (w/w) of a lubricant compound that may be composed by cellulose, glucose, polyethylene glycol and polyvinyl pyrrolidone (PVP) or a mixture thereof.

Effervescent tablets in general, when added to cold water, generate a gas which causes effervescence and produces a clear sparkling solution. The gas which gives the effervescence is always carbon dioxide which derives from the reaction between an acid and a base like carbonate or bicarbonate. The effervescent tablet consists of at least three components: the active ingredient; an acid; an alkali compound (basic ingredient) constituted by a carbonate or a bicarbonate. The acid and the alkali are the essential components which provide the effervescence and the disintegration of the tablet when is contacted with water.

As acidic component the citric acid both in the hydrated and anhydrous forms is more often used, but other edible acids like tartaric, fumaric, ascorbic, adipic and malic can be used as well. The carbonate, which represents the source of carbon dioxide which generates the effervescence, generally is a water-soluble alkaline carbonate.

Sodium bicarbonate is one of the most used carbonate because it is very soluble and of low cost. Alternatively, modified sodium bicarbonate can be used, obtained by heating common sodium bicarbonate in order to convert the surface of its particles to sodium carbonate so increasing its stability.

Other physiologically acceptable alkaline or alkaline earth metal carbonates may be used, such as potassium or calcium (bi)carbonate, sodium carbonate or sodium glycine carbonate.

Compositions of effervescent tablets may also include a lubricant which has to be necessarily selected from the totally water soluble compounds forming a clear solution. Examples of this kind of lubricants are sodium benzoate, sodium acetate, fumaric acid, polyethyleneglycols (PEG) higher than 4000, alanine and glycine.

The compositions of this invention may further comprise physiologically acceptable formulation excipients, such as those described in "Gennaro, Remington: The Science and Practice of Pharmacy" (20th Edition, 2000) (incorporated by reference into this patent, for example polyethylene glycol, carboxymethylcellulose, and/or hydroxypropyl methylcellulose. All such excipients and other ingredients preferably are (1) substantially pharmaceutically and/or veterinary pure and non-toxic in the amounts employed, and (2) compatible with the other ingredient(s). These excipients and other ingredients may be present in an amount of from a trace amount to about 20.0 percent (by weight). In some embodiments, the excipients and other ingredients are present in an amount of from a trace amount to about 10.0 percent (by weight).

Conventional excipients such as diluents, ligands, bufferings, sweeteners, flavourings, colorings, solubilizers, disintegrants, wetting agents and other excipients of common use may be added to the formulation. The direct compression of the simple physical blend of the components of the formulation represented an attempt to obviate the above technological difficulties. However such an operation has been carried out in controlled thermo-hygrometric conditions, for example at temperatures lower than 20-25°C and with relative humidity lower than 30%, using tabletting machines with tapered dies and punches faced with chromium alloys.

This objective is achieved by the tablet's preparation process through direct compression by a compaction method, through which before the compaction the sodium bicarbonate is dried on a dryer at 100° C during 1 hour, being then mixed with other ingredients, going through an 0.8mm mesh sieve and taken to a compressing machine where the tablet is produced always keeping the relative humidity of the environment at lower than 30% levels.

The current invention further provides a method of delivering a live vaccine to animals comprising
a. providing a quantity of drinking water for treating the animal,
b. mixing the composition according to the invention with said drinking water,
c. mixing a live vaccine in the drinking water mixture obtained through step b,
d. administering to the animal the live vaccine drinking water mixture obtained through step c.

In one embodiment the current invention provides a method of delivering a live vaccine to animals comprising:
a. providing a quantity of drinking water for treating the animal,
b. preparing a dry mix having a predetermined amount of a natural reducing agent, a buffer and a coloring agent and optionally additional additive,
c. forming a concentrated solution by adding a predetermined amount of the dry mix to a predetermined volume of water,
d. introducing the concentrated solution into the drinking water in a predetermined proportion that is sufficient for adjusting water quality for administration of water sanitizer sensitive medicaments or vaccines,
e. mixing the live vaccine in the drinking water mixture obtained through step d,
f. administering to the animal the live vaccine drinking water mixture obtained through step e.

In another embodiment the current invention provides a method of delivering a live vaccine to animals comprising:
a. providing a quantity of drinking water for treating the animal,
b. introducing an effervescent tablet having a predetermined amount of a natural reducing agent, a buffer and a coloring agent and optionally additional additive into the drinking water in a dosage that is sufficient for adjusting water quality for administration of water sanitizer sensitive medicaments or vaccines,
c. mixing the live vaccine in the drinking water mixture obtained through step b,
d. treating the animal with the live vaccine drinking water mixture obtained through step c.

In another embodiment the current invention provides a method of delivering a live vaccine to animals comprising:
a. providing a quantity of water,
b. introducing an effervescent tablet having a predetermined amount of a natural reducing agent, a buffer and a coloring agent and optionally additional additive into the water in a dosage that is sufficient for adjusting water quality for administration of water sanitizer sensitive medicaments or vaccines,
c. Introducing the concentrated solution into a larger volume of water to form a spray solution having a predetermined concentration of the natural reducing agent in the spray solution,
d. Adding the live vaccine to the spray solution, and
e. administering the spray solution to the animals.

One of the most critical aspects of ministering vaccines through drinking water is the time of permanence of the vaccine solution in the distribution system, because staying for long periods has a direct effect on the antigen's feasibility.

As an example, the vaccine virus of the Infectious Bronchitis may lose up to 50% of its feasibility after being diluted for 2 hours, while the Gumboro Disease virus can keep its almost total feasibility after 4 hours of dilution and the vaccine virus against Newcastle Disease can lose up to 50% of its feasibility on the first 30 minutes.

At the field level, the recommendation for time of consumption is of a minimum of 1 hour and a maximum of 2 hours, allowing balancing the access of a greater percentage of birds to the vaccine solution and the feasibility of the vaccine antigen in this solution. The current invention provides a composition that has maintains its effect after a long period of its reconstitution in water.

The composition according to the invention may be used to adjust water quality for administration of water sanitizer sensitive medicaments or vaccines to a human, a livestock animal e.g. sheep, cattle, pig, goat, poultry, a laboratory test animal, e.g. a rabbit, guinea pig, rat or mouse or a companion animal e.g. dog, cat or horse, a fish, shrimp or another aquatic animals or a wild animal. Especially preferred poultry species are chickens, also other poultry that is susceptible to one of the avian pathogens as turkeys, guinea fowl, ostrich, pigeons and partridges may be successfully vaccinated with the vaccine.

In general administration water sanitizer sensitive medicaments or vaccines is common practice in treating large numbers of poultry and pigs that are housed on large production farms. This type of application could also be used in aquaculture to treat fish or other aquatic animals. The composition according to the invention is also suitable for administration of water sanitizer sensitive medicaments or vaccines to individual animals.

### EXAMPLE 1

| Components | % (w/w) |
|---|---|
| Ascorbic Acid | 29 |
| Sodium Bicarbonate | 27 |
| Carboxymethylcellulose (CMC) | 11 |
| Citric Acid | 27 |
| Polyethylene glycol 6000 | 3 |
| FD & C Blue1 | 3 |

A tablet according to the invention is prepared through the following steps:
The Sodium Bicarbonate is placed on the dryer and kept during 1 hour at a temperature of 100 °C to eliminate the surface humidity.

Then, all the ingredients are fed into an adequate mixer, and mixed, according to the type of equipment. Afterwards, the mixture is poured and sieved through a mesh 0.8 mm sieve.

A tablet is formed through application of conventional compression techniques, keeping the environment's relative humidity lower than 30% and environment temperature lower than 25°C.

### EXAMPLE 2

In the same way as Example 1, we prepared the tablet illustrated below.

| Components | % (w/w) |
|---|---|
| Ascorbic Acid | 36 |
| Sodium Bicarbonate | 35 |
| Polyethylene glycol 6000 | 10.5 |
| Citric Acid | 10 |
| FD & C Blue 1 | 8.5 |

### EXAMPLE 3

The efficiency in adjusting water quality of the composition of to Example 1 was evaluated against three types of live viral vaccines for poultry manufactured by Akzo Nobel Ltda. - Intervet Division: Newcastle Disease (N.D. LaSota type), Infectious Bronchitis (I.B. Massachusetts type, strain Ma5) and Gumboro Disease (I.B.D.S. type D78).

The titration techniques used in this study follow the analytical procedure of the AVIPA Avicultura Integral e Patologia Animal S/C Ltda. laboratory registered at the Agriculture Ministry of Brazil. The titrations were made in embryos from a Specific Pathogen Free (SPF), with 9-day old or 11-day old embryonated eggs, through inoculation via allantoic cavity of the specific dilutions for each kind of vaccine.

The Embryo Infectious Dose (EID₅₀) for the Newcastle disease was based on the mortality and haemagglutination test (HA) of the live embryos after seven days of the inoculation.

For the Infectious Bronchitis and Gumboro Disease the EID₅₀ was calculated based on the type of embryo lesion after seven days of inoculation.

The effect of chlorine over the vaccine titration against the Newcastle Disease, Infectious Bronchitis and Gumboro Disease was evaluated at a level of water residual chlorine of 5 ppm.

Trichloroisocyanuric acid was used to prepare these solutions and the ratio of mass quantity of residual chlorine was kept constant and equal to 0.1 for the number of vaccine doses in all dilutions, representing with this the reality of what happens in the field during the reconstitution of the vaccine in the water used.

Table 2 shows that the composition of Example 1 when added to water containing a residual chlorine content of approximately 5 ppm, before the reconstitution of the vaccine virus, ensures that the vaccine titre against the Newcastle Disease, Infectious Bronchitis and Gumboro Disease remains unchanged, that is, the composition of Example 1 neutralizes the residual chlorine present in the water and does not interfere with the vaccine titration.

**Table 2**

| Characteristic | Newcastle Disease | Infectious Bronchitis | Gumboro Disease |
|---|---|---|---|
| Chlorine Concentration | 5.67 | 5.32 | 5.30 |
| Vaccine Doses/100 liters of drinking water | 5,000 | 5,000 | 5,000 |
| Minimum Titre to Agriculture Ministry of Brazil (log₁₀ EID ₅₀/dose) | 5.50 | 2.00 | 2.00 |
| Control Titre (log₁₀ EID₅₀ /dose) | 6.33 | 3.00 | 3.18 |
| Titre with Chlorine (log₁₀ EID₅₀ /dose) | 2.90 | 0 | 0 |
| Titre with Chlorine + composition of Example 1 (log₁₀ EID₅₀/dose) | 6.18 | 3.00 | 3.30 |

### EXAMPLE 4

The relative stability of the vaccine against the Newcastle Disease was evaluated during a period of 2 hours after the reconstitution in water. The titration was conducted for a live virus vaccine against the Newcastle Disease (N.D. LaSota type) manufactured by Akzo Nobel Ltda. - Intervet Division in the presence of the Example 1 composition and as comparison with the methods from the prior art: with powdered skim milk (P.S.M.) and alternatively the effervescent tablet CEVAMUNE produced by CEVA SANTE ANIMALE (WO 01/26622 A1).

As shown in table 3 the vaccine titre remained stable in both cases, that is, the product in question keeps the vaccine titration even after long period of its reconstitution in water.

**Table 3**

| **Time (hours)** | **log₁₀ EID₅₀/dose** | | |
|---|---|---|---|
| | Example 1 | CEVAMUNE | skim milk |
| 0 | 6.33 | 6.33 | 6.33 |
| 2 | 6.00 | 6.33 | 6.12 |

## Claims

1. Use of a composition comprising ascorbic acid or an alkali metal salt of ascorbic acid, a buffer and a coloring agent for adjusting water quality for administration of water sanitizer sensitive medicaments or vaccines.

2. The use according to claim 1 **characterized in that** the buffer is citric acid, alkali- or alkali earth bicarbonate and/or combinations thereof.

3. The use according to any preceding claims **characterized in that** the composition is in a solid form.

4. The use according to any preceding claims **characterized in that** the composition is in a tablet form.

5. The use according to claims 3 or 4 **characterized in that** the composition has the following generic composition:
1 to 40% (w/w) of ascorbic acid,
1 to 40% (w/w) of sodium bicarbonate,
1 to 30% (w/w) of citric acid,
1 to 35% (w/w) of a food grade coloring agent,
1 to 20% (w/w) of a lubricant compound.

6. The use according to any of the preceding claims **characterized in that** the composition is a effervescent tablet

7. The use according to claims 1 or 2 **characterized in that** the composition is in a liquid form.

8. A method of de-chlorinating water for administering of water sanitizer sensitive medicaments or vaccines to animals comprising:
a. mixing the water-sanitizer according to claim 1 with drinking water,
b. mixing a water sanitizer sensitive medicament or vaccine in the drinking water mixture obtained through step a,

9. A method of de-chlorinating water for administering of water sanitizer sensitive medicaments or vaccines to animals comprising:
a. preparing a dry mix having a predetermined amount of ascorbic acid , a buffer and a coloring agent and optionally additional additives,
b. forming a concentrated solution by adding a predetermined amount of the dry mix obtained through step a) to a predetermined volume of water,
c. introducing the concentrated aqueous solution obtained through step b) into the drinking water in a predetermined proportion that is sufficient for adjusting water quality for administration of water sanitizer sensitive medicaments or vaccines,
d. mixing the water sanitizer sensitive medicaments or vaccine in the drinking water mixture obtained through step c),

## Patentansprüche

1. Verwendung einer Zusammensetzung umfassend Ascorbinsäure oder ein Alkalimetallsalz der Ascorbinsäure, einen Puffer und ein Farbmittel zum Anpassen der Wasserqualität für die Verabreichung von Medikamenten oder Impfstoffen, die auf Wasserdesinfektionsmittel empfindlich reagieren.

2. Verwendung nach Anspruch 1, **dadurch gekennzeichnet, dass** es sich bei dem Puffer um Zitronensäure, Alkali- oder Erdalkalihydrogencarbonat und/oder Kombinationen davon handelt.

3. Verwendung nach einem der vorhergehenden Ansprüche, **dadurch gekennzeichnet, dass** die Zusammensetzung in fester Form vorliegt.

4. Verwendung nach einem der vorhergehenden Ansprüche, **dadurch gekennzeichnet, dass** die Zusammensetzung in Tablettenform vorliegt.

5. Verwendung nach den Ansprüchen 3 oder 4, **dadurch gekennzeichnet, dass** die Zusammensetzung die folgende allgemeine Zusammensetzung aufweist:
1 bis 40% (w/w) Ascorbinsäure,
1 bis 40% (w/w) Natriumhydrogencarbonat,
1 bis 30% (w/w) Zitronensäure,
1 bis 35% (w/w) eines Farbmittels in Lebensmittelqualität,
1 bis 20% (w/w) einer Gleitmittelverbindung.

6. Verwendung nach einem der vorhergehenden Ansprüche, **dadurch gekennzeichnet, dass** die Zusammensetzung eine Brausetablette ist.

7. Verwendung nach den Ansprüchen 1 oder 2, **dadurch gekennzeichnet, dass** die Zusammensetzung in flüssiger Form vorliegt.

8. Verfahren zum Entchloren von Wasser zum Verabreichen von Medikamenten oder Impfstoffen, die gegen Wasserdesinfektionsmittel empfindlich sind, an Tiere, das Folgendes umfasst:
a. Vermischen des Wasserdesinfektionsmittels nach Anspruch 1 mit Trinkwasser,
b. Einmischen eines Arzneimittels oder Impfstoffs, das/der gegenüber Wasserdesinfektionsmitteln empfindlich ist, in die in Schritt a erhaltene Trinkwassermischung.

9. Verfahren zum Entchloren von Wasser zum Verabreichen von Medikamenten oder Impfstoffen, die gegen Wasserdesinfektionsmittel empfindlich sind, an Tiere, das Folgendes umfasst:
a. Herstellen einer Trockenmischung mit einer vorbestimmten Menge an Ascorbinsäure, eines Puffers und eines Farbmittels und gegebenenfalls zusätzlichen Zusatzstoffen,
b. Bilden einer konzentrierten Lösung durch Hinzufügen einer vorbestimmten Menge der im Schritt a) erhaltenen Trockenmischung zu einem vorbestimmten Wasservolumen,
c. Einbringen der in Schritt b) erhaltenen konzentrierten wässrigen Lösung in das Trinkwasser in einem vorbestimmten Anteil, der ausreicht, um die Wasserqualität an die Verabreichung von Medikamenten oder Impfstoffen, die gegenüber Wasserdesinfektionsmitteln empfindlich sind, anzupassen,
d. Einmischen der Medikamente oder der Impfstoffen, die/der auf Wasserdesinfektionsmitte empfindlich reagieren, in die in Schritt c) erhaltene Trinkwassermischung.

## Revendications

1. Utilisation d'une composition comprenant de l'acide ascorbique ou un sel de métal alcalin d'acide ascorbique, un tampon et un agent colorant, afin d'ajuster la qualité de l'eau pour une administration de médicaments ou de vaccins sensibles à un agent d'assainissement de l'eau.

2. Utilisation selon la revendication 1, **caractérisée en ce que** le tampon est l'acide citrique, un bicarbonate alcalin ou alcalino-terreux et/ou des combinaisons de ceux-ci.

3. Utilisation selon l'une quelconque des revendications précédentes, **caractérisée en ce que** la composition se présente sous une forme solide.

4. Utilisation selon l'une quelconque des revendications précédentes, **caractérisée en ce que** la composition se présente sous une forme de comprimé.

5. Utilisation selon les revendications 3 ou 4, **caractérisée en ce que** la composition présente la composition générique suivante :
de 1 à 40% (p/p) d'acide ascorbique,
de 1 à 40% (p/p) de bicarbonate de sodium,
de 1 à 30% (p/p) d'acide citrique,
de 1 à 35% (p/p) d'un agent colorant de qualité alimentaire,
de 1 à 20% (p/p) d'un composé lubrifiant.

6. Utilisation selon l'une quelconque des revendications précédentes, **caractérisée en ce que** la composition est un comprimé effervescent.

7. Utilisation selon les revendications 1 ou 2, **caractérisée en ce que** la composition se présente sous une forme liquide.

8. Méthode de déchloration de l'eau, pour une administration de médicaments ou de vaccins sensibles à un agent d'assainissement de l'eau, à des animaux, comprenant :
a. le mélange de l'agent d'assainissement de l'eau selon la revendication 1 avec de l'eau de boisson,
b. le mélange d'un médicament ou d'un vaccin sensible à un agent d'assainissement de l'eau dans le mélange d'eau de boisson obtenu par l'intermédiaire de l'étape a.

9. Méthode de déchloration de l'eau, pour une administration de médicaments ou de vaccins sensibles à un agent d'assainissement de l'eau, à des animaux, comprenant :
a. la préparation d'un mélange sec ayant une quantité prédéterminée d'acide ascorbique, d'un tampon et d'un agent colorant et éventuellement des additifs supplémentaires,
b. la formation d'une solution concentrée par l'addition d'une quantité prédéterminée du mélange sec obtenu par l'intermédiaire de l'étape a) à un volume prédéterminé d'eau,
c. l'introduction de la solution aqueuse concentrée obtenue par l'intermédiaire de l'étape b) dans l'eau de boisson selon une proportion prédéterminée qui est suffisante pour ajuster la qualité de l'eau pour une administration de médicaments ou de vaccins sensibles à un agent d'assainissement de l'eau,
d. le mélange des médicaments ou vaccins sensibles à un agent d'assainissement de l'eau dans le mélange d'eau de boisson obtenu par l'intermédiaire de l'étape c) .
